# EUROPÄISCHE PATENTSCHRIFT

(11) **EP 0 352 601 B1**
(45) Veröffentlichungstag und Bekanntmachung des Hinweises auf die Patenterteilung: **19.10.1994**
(21) Anmeldenummer: 89113138.5
(22) Anmeldetag: 18.07.1989
(51) Int. Cl.: A61B 19/00, A61B 19/08

(54) **Schutzschild, insbesondere für transurethrale Eingriffe beim Mann**
Schield particularly for male transurethral operation
Ecran de protection en particulier pour une opération transurétrale chez l'homme

(30) Priorität: 29.07.1988 DE 8809674 U
(43) Veröffentlichungstag der Anmeldung: 31.01.1990
(73) Patentinhaber: Fresenius AG, 61350 Bad Homburg (DE)
(72) Erfinder: Müller, Bernd, D-3150 Peine (DE)
(74) Vertreter: Fuchs Mehler Weiss

(56) Entgegenhaltungen:
- EP-A- 0 182 766
- US-A- 4 471 769

## Beschreibung

Die Neuerung betrifft ein Schutzschild, insbesondere für transurethrale Eingriffe beim Mann, mit einer vorzugsweise aus Kunststoff hergestellten Folie die eine erste Ausnehmung aufweist, auf der ein kondomähnlicher Schlauch aus vorzugsweise Latex oder ähnlichem Material angeordnet ist, wobei die Folie eine zweite Ausnehmung aufweist.

Derartige Schutzschilder, wie z.B. bekannt aus EP-A-0 182 766, werden im medizinischen Bereich zur Rektaluntersuchung und auch bei transurethralen Resektionen eingesetzt. Bei diesen transurethralen Eingriffen wird vom Untersucher bzw. Operateur durch die Urethra ein Instrument eingeführt, um die Urethra und die Blase optisch darzustellen. Der Arzt schaut zu diesem Zweck durch eine durch das Instrument eingeführte Optik und ist somit in unmittelbarem Augen- und Schleimhautkontakt mit den eingesetzten Spüllösungen. Kommt es bei dieser Untersuchung des Patienten zu Husten oder sonstigen plötzlichen Muskelkontraktionen der Blase, so wird bedingt durch den plötzlichen intravesikalen Druckanstieg das Blasenfüllmedium (Spülflüssigkeit) zwischen Arbeitselement und Urethra-Schleimhaut herausgepreßt. Bedingt durch den sehr geringen Abstand zwischen dem Genitalorgan des Patienten und dem Gesicht des Operateurs besteht die Gefahr der Kontamination von Schleimhäuten des Operateurs. Besonders gefährlich ist die Situation bei infizierten Patienten, insbesondere Hepatitis infizierten Patienten und AIDS-Kranken.

Der bisher verwendete Schutzschild ist als sogenannter Rektalschild ausgebildet und weist eine Folie auf, die aus einem sehr weichen Kunststoff hergestellt ist. Die Folie ist groß genug, um einen weiten Bereich um das Genitalteil des Mannes abzudecken. Im Genitalbereich selbst ist die Folie mit einer Ausnehmung versehen, auf der ein kondomähnlicher Schlauch, der vorzugsweise aus Latex oder einem ähnlichen Material besteht, mittels einer Manschette auf der Abdeckfolie befestigt ist. Ferner weist der bekannte Schutzschild eine zweite Ausnehmung auf, durch die bei der Operation das Glied und das Skrotum des Mannes hindurchgeführt werden kann.

Der bekannte Schutz- bzw. Rektalschild wird bei transurethralen Eingriffen am Patienten angelegt und er wird dazu benutzt, um bei Resektion dem Operateur die Möglichkeit zu geben, rektal die Prostata so anzuheben, daß der prostatische Bereich der Harnröhre besser ausrestiziert werden kann. Aus diesem Grund wird das am Rektalschild befindliche Kondom vom Operateur rektal eingeführt, so daß der Operateur mit seinem sterilen Handschuh rektal digitalisieren kann, ohne dabei seine sterile Hand zu kontaminieren.

Da jedoch das Glied des Mannes durch die zweite Öffnung im bekannten Schutzschild hindurchgeführt ist, ist der Operateur bei der Arbeit vor austretender Spülflüssigkeit aufgrund der eingangs erläuterten Problematik nicht geschützt.

Es ist daher Aufgabe der Neuerung, einen Schutzschild der im Oberbegriff des Anspruchs 1 angegebenen Art zu schaffen, der es möglich macht, den Operateur vor durch plötzlich auftretende intravesikaler Druckerhöhung bedingte Flüssigkeitsaustritte aus der Urethra zu schützen.

Die Lösung dieser Aufgabe erfolgt durch die Merkmale des Anspruches 1.

Dadurch wird ermöglicht, daß als Schutz zwischen Gesicht und Schleimhäuten des Operateurs der zweite kondomartige Schlauch über das männliche Genitalorgan gestülpt werden kann, wobei dieser Schlauch an seiner vorderen Spitze entweder eine Öffnung zur Einführung des Instrumentenschaftes aufweist, deren Ränder sich dichtlippenartig an den Instrumentenschaft anlegen oder der mit einem Dichtelement an seiner Spitze versehen ist, wobei bei dieser Ausführung dann die Abdichtung durch ein enges Anlegen des Dichtelementes an den Instrumentenschaft erfolgt.

Dieses Dichtelement, das schwamm- oder membranartig ausgebildet sein kann, wird vor Beginn der Operation mit Desinfektionslösung getränkt und bildet so eine sichere Barriere für aus der Urethra austretende Flüssigkeiten.

Das neuerungsgemäße Schutzschild stellt somit einen universell an das männliche Genitalorgan adaptierbaren Schutz dar, der selbst bei plötzlich auftretender intravesikaler Druckerhöhung ein Kontaminieren des Gesichtes und der Schleimhäute des Operateurs gänzlich ausschließt.

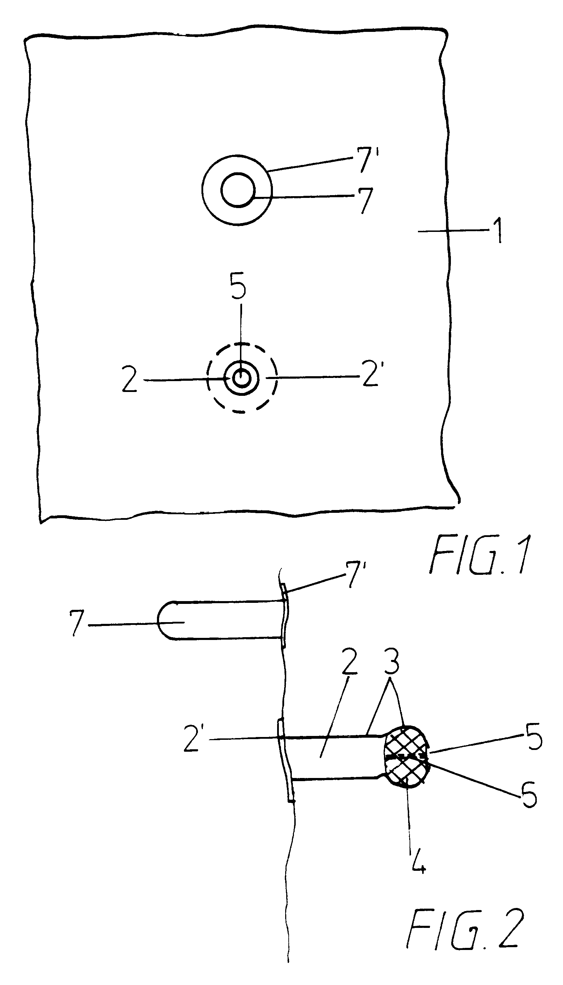

An einem Ausführungsbeispiel soll die Neuerung näher erläutert werden. Die beiliegende Zeichnung zeigt in Figur 1 die Vorderansicht des neuerungsgemäßen Schutzschildes und Figur 2 eine Seitenansicht des erfindungsgemäßen Schutzschildes.

Wie aus den beiliegenden Zeichnungsfiguren zu erkennen ist, besteht der neuerungsgemäße Schutzschild aus einer Folie 1, mit ausreichend großer Fläche, um den Genitalbereich des Patienten großflächig zu überdecken. Diese Folie 1 hat vorzugsweise eine rechteckige Grundform und besteht aus einem äußerst weichem Kunststoff, so daß sich die Folie 1 weich an den Körper des Patienten anlegen kann. Dadurch wird der Patient vor unnötiger Benetzung mit bei transurethralen Eingriffen benutzter Spüllösung geschützt.

In ihrem zentralen Bereich oder in ihrem unteren Bereich weist die Folie 1 einen kondomähnlichen Schlauch 2 auf, der aus Latex oder einem ähnlichen weichen Material besteht. In einer vorzugsweisen Ausführungsform ist das hintere offene Ende des kondomähnlichen Schlauches 2 mittels einer Manschette 2′ auf der Kunststoffolie 1 aufgebracht. Dies kann beispielsweise mittels Klebung, Schweißung oder Vulkanisieren erfolgen. Es ist aber auch möglich, daß die Folie aus dem gleichen Material wie der kondomähnliche Schlauch besteht, so daß diese Teile als eine Einheit ausgebildet sind.

Der kondomähnliche Schlauch 2 ist dabei anatomisch so angepaßt, daß er zur Aufnahme des männlichen Gliedes dient.

In seinem vorderen Bereich des kondomähnlichen Schlauches 2 weist dieser in einer besonderen Ausbildungsform eine abakusartige Ausformung 3 auf. In dieser Ausformung 3 ist ein Dichtelement 4 eingebracht, daß als Schwämmchen oder saugfähige Membran o.dgl. ausgebildet ist. Dieses Dichtelement 4 wird vor Beginn der Operation mit Desinfektionsmittel getränkt und bildet somit eine sichere Barriere für aus der Urethra austretende Flüssigkeiten. Andererseits kann jedoch bereits werkseitig das Dichtelement 4 mit dem Desinfektionsmittel versehen werden, so daß eine bereits gebrauchsfertige Anordnung zum Einsatz kommen kann.

Die abakusartige Ausformung 3 braucht in einer anderen Ausführungsform nicht ausdrücklich in den kondomartigen Schlauch eingeformt zu sein, vielmehr wird eine leichte abakusartige Ausformung auch dadurch gebildet, daß das Dichtelement 4 in den vorderen Bereich des kondomähnlichen Schlauches 2 eingebracht ist und der geringfügig größere Durchmesser des Dichtelementes 4 die Wandung des kondomähnlichen Schlauches 2 abakusartig nach außen ausformt. Bedingt durch die Rückstellkraft des kondomähnlichen Schlauches 2 wird das Dichtelement 4 im Schlauch 2 gehalten.

Das vordere Ende des kondomähnlichen Schlauches 2 ist in seinem zentralen Bereich mit einer Öffnung 5 versehen, die einen etwa gleichen oder geringfügig kleineren Durchmesser aufweist, als das einzuführende Operationsinstrument. Dadurch wird das mit Desinfektionslösung getränkte Dichtelement 4 beim Einführen des Operationsinstrumentes gegenüber der Umgebung abgedichtet, da der Rand der Öffnung 5 benachbart bzw. eng anliegend an dem Schaft des Operationsinstrumentes angeordnet ist. Es ist auch in einer anderen Ausbildungsform möglich, die vordere Spitze des kondomähnlichen Schlauches 2 zur Formstabilisierung der Eingangsöffnung 5 offenzurollen oder im Bereich der Öffnung 5 einen Verstärkungsring einzubringen bzw. das Material des kondomähnlichen Schlauches 2 verstärkt auszubilden. Das in der abakusartigen Ausformung 3 befindliche Dichtelement kann vorteilhafterweise zentrisch geschlitzt sein bzw. mit einer sich selbsttätig schließenden Durchgangsöffnung 6 versehen, so daß es leicht mit dem Operationsinstrument zu durchstechen ist. Dabei legt sich die Durchgangsöffnung weich um das vorzugsweise zu transurethralen Eingriffen eingeführte Operationsinstrument.

Wie Figur 1 ferner zeigt, ist neben dem kondomähnlichen Schlauch 2, der an seiner dem Dichtelement 4 gegenüberliegenden Stelle offen ist und dort eine in der Folie 1 vorhandene Öffnung übergreift, ein weiterer kondomähnlicher Schlauch 7 in einem Bereich der Folie 1 angeordnet, der bei einem Eingriff eine rektale Digitalisierung durch Eingreifen des Operateurs in diesen Schlauch 7 ermöglicht. Der Schlauch 7 ist im Gegensatz zum Schlauch 2 an seiner Vorderseite geschlossen und an seiner offenen Seite ebenfalls über eine Manschette 7′ an der Folie 1 derart angebracht, daß er auch eine dort vorgesehene Ausnehmung übergreift. Diese in Figur 1 nicht näher dargestellte Ausnehmung ermöglicht es dem Operateur also, die Folie 1 zu durchgreifen und damit durch die genannte Ausbildung in den Innenraum des Schlauches 7 zu gelangen.

Beim Schlauch 2 ist diese Ausnehmung in der Folie 1 erforderlich, damit das durch den Schlauch 2 eingeführte Instrument die Folie 1 durchdringen kann.

Zur Ausbildung des Schlauches 2 ist ferner zu sagen, daß dieser alternativ zum Vorsehen eines Dichtelementes mit einer Öffnung in seiner Spitze versehen sein kann, die derart groß dimensioniert werden kann, daß ihre Ränder bzw. ihr Rand sich nach Art einer Dichtlippe an den eingeführten Instrumentenschaft anlegt und somit das Austreten von Spülflüssigkeit oder anderen Flüssigkeiten verhindern kann.

Das Schutzschild stellt somit einen universell an das männliche Genitalorgan adaptierbaren Schutz dar, und schließt selbst bei plötzlich austretender intravesikaler Druckerhöhung ein Kontaminieren des Gesichts und der Schleimhäute des Operateurs aus.

## Patentansprüche

1. Schutzschild, insbesondere für transurethrale Eingriffe beim Mann, mit einer vorzugsweise aus Kunststoff hergestellten Folie (1), die eine erste Ausnehmung aufweist, auf der ein kondomähnlicher Schlauch (7) aus vorzugsweise Latex oder ähnlichem Material angeordnet ist, wobei die Folie eine zweite Ausnehmung aufweist, dadurch gekennzeichnet, daß auf der zweiten Ausnehmung ein weiterer kondomähnlicher Schlauch (2) angeordnet ist, der an seiner Spitze eine Öffnung (5) zum Einführen von Instrumenten aufweist.

2. Schutzschild nach Anspruch 1, dadurch gekennzeichnet, daß die Öffnung an der Spitze des kondomähnlichen Schlauches (2) dichtlippenartige Ränder aufweist, die sich an den Instrumentenschaft anlegen.

3. Schutzschild nach Anspruch 1, dadurch gekennzeichnet, daß der zweite kondomähnliche Schlauch (2) in seinem vorderen Bereich (3) ein mit einer Durchgangsöffnung (6) versehenes Dichtelement (4) aufweist, daß durch den kondomartigen Schlauch (2) gehalten ist.

4. Schutzschild nach Anspruch 3, dadurch gekennzeichnet, daß die Öffnung (5) an der Spitze des kondomartigen Schlauches (2) einen geringfügig kleineren Durchmesser aufweist, als das einzuführende Instrument.

5. Schutzschild nach Anspruch 3 oder 4, dadurch gekennzeichnet, daß das Dichtelement (4) ein Schwämmchen oder eine saugfähige Membran ist.

6. Schutzschild nach einem der Ansprüche 1 bis 5, dadurch gekennzeichnet, daß der kondomähnliche Schlauch (2) eine derartige anatomische Form aufweist, daß er zur Aufnahme des männlichen Gliedes geeignet ist.

7. Schutzschild nach einem der Ansprüche 2 bis 6, dadurch gekennzeichnet, daß die Schlauchspitze eine abakusartige Ausformung (3) zur Aufnahme des Dichtelementes (4) aufweist.

## Claims

1. Shield, particularly for male transurethral operation, having a foil (1) preferably made of synthetic material and comprising a first aperture on which there is disposed a condom-like tube (7) preferably made of latex or a similar material, said foil comprising a second aperture, characterized in that an additional condom-like tube (2) is disposed on the second aperture, having an opening (5) at its tip for the introduction of instruments.

2. Shield according to claim 1, characterized in that the opening at the tip of the condom-like tube (2) comprises sealing lip-like borders that snuggle up to the shaft of the instrument.

3. Shield according to claim 1, characterized in that the second condom-like tube (2) comprises a sealing element (4) in its forepart (3), said sealing element being equipped with a connecting passage (6) and being fixed by the condom-like tube (2).

4. Shield according to claim 3, characterized in that the opening (5) at the tip of the condom-like tube (2) has a slightly smaller diameter than the instrument to be introduced.

5. Shield according to claim 3 or 4, characterized in that the sealing element (4) is a small sponge or an absorbent membrane.

6. Shield according to one of claims 1 through 5, characterized in that the condom-like tube (2) comprises such an anatomic form that it is suitable to accept the male member.

7. Shield according to one of claims 2 through 6, characterized in that the tip of the tube has the form of an abacus (3) to accept the sealing element (4).

## Revendications

1. Ecran de protection, en particulier pour une opération transurétrale chez l'homme, ayant de préférence une feuille (1) en matière artificielle et présentant un premier trou sur lequel on a arrangé un tuyeau (7) ressemblant à un condom, de préférence en latex ou en matières semblables, la feuille comportant un second trou, caractérisé en ce que, sur ledit second trou, on a disposé un autre tuyeau (2) ressemblant à un condom qui présente à son bout un orifice (5) pour l'introduction d'instruments.

2. Ecran de protection d'après la revendication 1, caractérisé en ce que l'orifice au bout du tuyeau ressemblant à un condom (2) présente des bordures ressemblant à une lèvre d'étanchéité qui se serrent contre la hampe de l'instrument.

3. Ecran de protection d'après la revendication 1, caractérisé en ce que le deuxième tuyeau ressemblant à un condom (2) présente à son devant (3) un élément d'étanchéité (4) pourvu d'un orifice de (6) passage et arrêté par le tuyeau (2).

4. Ecran de protection d'après la revendication 3, caractérisé en ce que l'orifice (5) au bout du tuyeau ressemblant à un condom (2) présente un diamètre légèrement moindre que celui de l'instrument que l'on veut introduire.

5. Ecran de protection d'après les revendications 3 ou 4, caractérisé en ce que l'élément d'étanchéité (4) est une petite éponge ou une membrane absorbeuse.

6. Ecran de protection d'après une des revendications 1 à 5, caractérisé en ce que le tuyeau ressemblant à un condom (2) présente une telle forme anatomique, qu'il convient à la réception du membre viril.

7. Ecran de protection d'après une des revendications 2 à 6, caractérisé en ce que le bout du tuyeau présente une conformation en forme d'un abaque (3) pour la réception de l'élément d'étanchéité.
